(19) Europäisches Patentamt
European Patent Office
Office européen des brevets

(11) **EP 4 597 082 A1**

(12) **EUROPEAN PATENT APPLICATION**
published in accordance with Art. 153(4) EPC

(43) Date of publication:
**06.08.2025 Bulletin 2025/32**

(21) Application number: 23879435.8

(22) Date of filing: **07.08.2023**

(51) International Patent Classification (IPC):
**G01N 21/84** *(2006.01)*    **G01N 21/89** *(2006.01)*

(52) Cooperative Patent Classification (CPC):
**G01N 21/84; G01N 21/89**

(86) International application number:
**PCT/JP2023/028774**

(87) International publication number:
**WO 2024/084782 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.10.2022 JP 2022166258**

(71) Applicant: Hamamatsu Photonics K.K.
Hamamatsu-shi, Shizuoka 435-8558 (JP)

(72) Inventors:
• KONDO Hideyuki
  Hamamatsu-shi, Shizuoka 435-8558 (JP)
• MATSUDA Shunsuke
  Hamamatsu-shi, Shizuoka 435-8558 (JP)
• TSUCHIYA Kunihiko
  Hamamatsu-shi, Shizuoka 435-8558 (JP)

(74) Representative: Hoffmann Eitle
Patent- und Rechtsanwälte PartmbB
Arabellastraße 30
81925 München (DE)

(54) **IMAGE ACQUISITION DEVICE, INSPECTION DEVICE, AND IMAGE ACQUISITION METHOD**

(57)     An image acquisition device 1 includes an illumination device 2 that irradiates a target object S with light from a range of a light irradiation unit 2a, and an imaging device 7 that detects light that is specularly reflected by the target object S in the light, via an imaging lens 7a, in which a straight line connecting a center of the light irradiation unit 2a and an intersection between an optical axis of the imaging lens 7a and the target object S is set to be inclined by 2 degrees or more and 120 degrees or less with respect to the optical axis, and a solid angle of the light irradiation unit 2a as viewed from the intersection is set to 0 steradians or more and 0.15 steradians or less.

*Fig.3*

EP 4 597 082 A1

## Description

## Technical Field

One aspect of an embodiment relates to an image acquisition device, an inspection device, and an image acquisition method.

## Background Art

In the related art, a device has been known, which inspects an inspection target object by detecting light with which the inspection target object is irradiated. For example, in a device disclosed in Patent Literature 1, an imaging unit detects transmitted light transmitted through an inspection target object and reflected light reflected by the inspection target object, and inspects the inspection target object based on detection data output from the imaging unit.

## Citation List

## Patent Literature

[Patent Literature 1] Japanese Unexamined Patent Publication No. 2021-189071

## Summary of Invention

## Technical Problem

Depending on the detection data acquired by the related-art device described above, it tends to be difficult to detect an object having a property of reflecting the light in the inspection target object. Therefore, it is required to acquire data that enables efficient detection of the object having the property of reflecting the light on the inspection target object.

Therefore, one aspect of the embodiment is made in view of such an issue, and an object of the aspect is to provide an image acquisition device, an inspection device, and an image acquisition method, with which image data that enables efficient detection of an object present on a target object and having a property of reflecting the light can be acquired.

## Solution to Problem

A first aspect of the embodiment relates to an image acquisition device including: a light irradiation device that irradiates a target object with light from a range of a light irradiation unit; and an imaging device that detects light that is specularly reflected by the target object in the light, via an imaging lens, in which a straight line connecting a center of the light irradiation unit and an intersection between an optical axis of the imaging lens and the target object is set to be inclined by 2 degrees or more and 120 degrees or less with respect to the optical axis, and a solid angle of the light irradiation unit as viewed from the intersection is set to 0 steradians or more and 0.15 steradians or less.

Alternatively, a second aspect of the embodiment relates to an image acquisition method including: a light irradiation step of using a light irradiation device to irradiate a target object with light from a range of a light irradiation unit; and an imaging step of using an imaging device to detect light that is specularly reflected by the target object in the light, via an imaging lens, in which a straight line connecting a center of the light irradiation unit and an intersection between an optical axis of the imaging lens and the target object is set to be inclined by 2 degrees or more and 120 degrees or less with respect to the optical axis, and a solid angle of the light irradiation unit as viewed from the intersection is set to 0 steradians or more and 0.15 steradians or less.

According to the first aspect or the second aspect, in a case in which an object having a property of reflecting the light is present on the target object, it is possible to cause the specularly reflected light from the object to be efficiently incident on the imaging device via the imaging lens, and it is possible to make the intensity of the specularly reflected light incident from the object sufficiently larger than the intensity of diffused light incident from the target object. In particular, by setting the solid angle of the light irradiation unit as viewed from the intersection between the optical axis of the imaging lens and the target object to 0 steradians or more and 0.15 steradians or less, a ratio between the intensity of the specularly reflected light and the intensity of the diffused light can be non-linearly increased. As a result, it is possible to acquire the image data that enables efficient detection of the object present on the target object and having the property of reflecting the light.

Alternatively, a third aspect of the embodiment relates to an inspection device including: the image acquisition device according to any one of the first aspect; a transport device that transports the target object in a predetermined direction; and an inspection processing unit that inspects the target object based on data output from the image acquisition device.

According to the third aspect, it is possible to efficiently detect the objects present on a plurality of target objects and having the property of reflecting the light while transporting the plurality of target objects.

## Advantageous Effects of Invention

According to any aspect of the present invention, it is possible to acquire the image data that enables the efficient detection of the object present on the target object and having the property of reflecting the light.

## Brief Description of Drawings

Fig. 1 is a schematic configuration diagram illustrating an image acquisition device 1 according to an embodiment.

Fig. 2 is a diagram illustrating an image of reflected light generated in a target object S in a case in which the image acquisition device 1 of Fig. 1 is used.

Fig. 3 is a diagram illustrating disposition of an illumination device 2 with respect to an imaging device 7 and a light emission range of the illumination device 2 in the image acquisition device 1.

Fig. 4 is a graph illustrating a relationship between a solid angle ω and a signal ratio representing detection accuracy in the image acquisition device 1.

Fig. 5 is a graph illustrating a relationship between the solid angle ω and the signal ratio representing the detection accuracy in the image acquisition device 1.

Fig. 6 is a schematic configuration diagram illustrating an inspection system 100 according to the embodiment.

Fig. 7 is a flowchart illustrating a procedure of an inspection method for a target object S using the inspection system 100.

Fig. 8 is a schematic configuration diagram illustrating an image acquisition device 1A according to a modification example.

Fig. 9 is a schematic configuration diagram illustrating an image acquisition device 1B according to a modification example.

Fig. 10 is a schematic configuration diagram illustrating an image acquisition device 1C according to a modification example.

Fig. 11 is a diagram illustrating image data acquired by the image acquisition device 1B according to the modification example.

Fig. 12 is a diagram illustrating image data acquired by an image acquisition device according to a comparative example.

**Description of Embodiments**

Hereinafter, embodiments of the present invention will be described in detail with reference to the accompanying drawings.

In the description, the same elements or the elements having the same functions are denoted by the same reference numerals, and the duplicate description will be omitted.

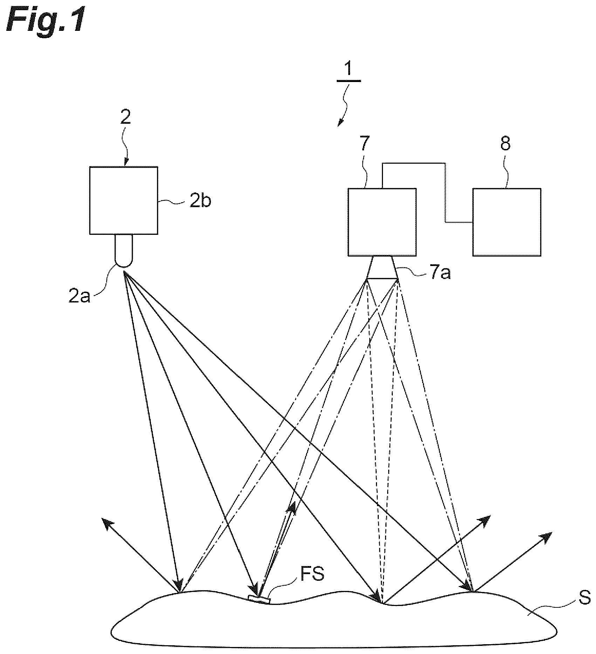

Fig. 1 is a schematic configuration diagram illustrating an image acquisition device 1 according to the embodiment. The image acquisition device 1 is a device that acquires image data of a target object for the purpose of inspecting whether or not a foreign substance is present on the target object such as food. However, the target object, which is a target of an inspection performed by the image acquisition device 1, may be other articles such as electronic components, in addition to food represented by beef, pork, chicken, lamb, and processed food.

The image acquisition device 1 includes an illumination device (light irradiation device) 2, an imaging device 7, and an image processing device 8. Hereinafter, each component of the image acquisition device 1 will be described in detail.

The illumination device 2 is configured by a light irradiation unit 2a that performs the irradiation with light and a body portion 2b in which a lighting circuit for lighting a light emitting element in the light irradiation unit 2a is incorporated, and performs irradiation by diffusing the light toward the target object S. Examples of the light emitting element incorporated in the light irradiation unit 2a include an LED, a superluminescent diode (SLD), a laser, and a halogen lamp. The light irradiation unit 2a has a configuration in which the light emitting element that is one or a plurality of point light sources is incorporated, and irradiation can be performed by diffusing the light from a point-like light emission range of the light irradiation unit 2a through a lens, a diffusion plate, and the like. A shape of the light emission range of the light irradiation unit 2a may be a planar shape or may be a curved shape such as a spherical shape.

The imaging device 7 is a device that is disposed at a position at which reflected light generated by specularly reflecting the light emitted from the illumination device 2 by the target object S can be detected, and detects a two-dimensional image of the light including the reflected light to acquire image data. As the imaging device 7, a complementary metal-oxide-semiconductor (CMOS) camera, a charge-coupled device (CCD) camera, or the like is used. In a case in which the target object S is transported in a predetermined direction by a transport device, a line sensor camera or a time delay integration (TDI) sensor camera may be used as the imaging device 7.

The imaging device 7 includes an imaging lens 7a. The imaging lens 7a forms an image of the light including the reflected light from the target object S, as the two-dimensional image, on a light receiving surface (not illustrated) of an imaging element inside the imaging device 7. The imaging device 7 detects the light including the reflected light from the target object S via an image-forming lens.

The image processing device 8 is a device that detects a foreign substance on the target object S by receiving the image data acquired by the imaging device 7. Physically, the image processing device 8 is a computing device (computer or the like) that incorporates a central processing unit (CPU) or a graphics processing unit (GPU) as a processor, a random-access memory (RAM) or a read-only memory (ROM) as a recording medium, a communication module, an input/output module, and the like. In addition, the image processing device 8 may be configured by a field programmable gate array (FPGA) or an application-specific integrated circuit (ASIC). The image processing device 8 may acquire the image data from the imaging device 7 via a cable, or may acquire the image data from the imaging device 7 by wireless communication.

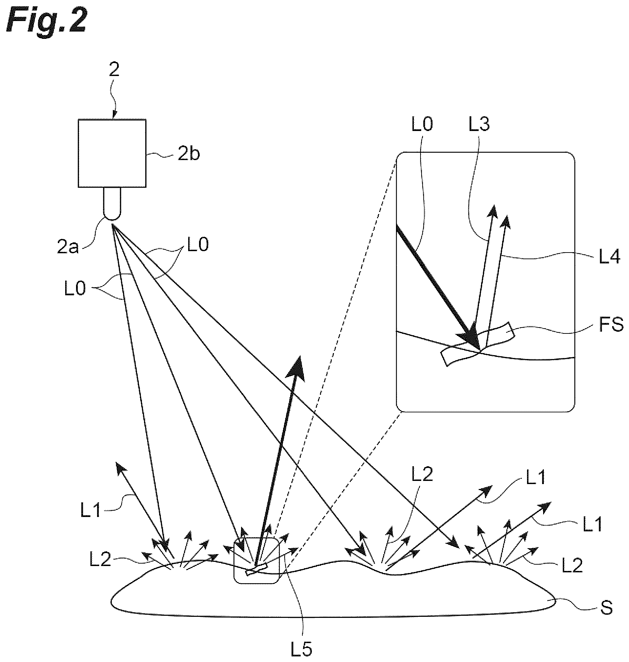

Here, a mechanism for detecting reflected light from the target object S in the image acquisition device 1 will be described with reference to Fig. 2. Fig. 2 is a diagram illustrating an image of the reflected light generated in the target object S in a case in which the image acquisition device 1 is used.

Each ray L0 of the light diffused by the illumination device 2 for irradiation widely reaches a surface of the target object S. The target object S such as food has a property of generating specularly reflected light L1 and diffusely reflected light L2 in a case in which the light is incident. In addition, in a case in which a foreign substance FS such as a plastic film that is an object having a property of transmitting the light is present on the surface of the target object S, the ray L0 of the light incident on the foreign substance FS causes a specularly reflected light L3 that is specularly reflected on the surface of the foreign substance FS, a specularly reflected light L4 that is transmitted through the foreign substance FS and then specularly reflected on a rear surface of the foreign substance FS, and a diffusely reflected light L5 that is diffusely reflected by the foreign substance FS. In this case, the intensity of the specularly reflected light L3 and L4 is relatively higher than the intensity of the specularly reflected light L1, and the intensity of the specularly reflected light L3 and L4 is relatively higher than the intensity of the diffusely reflected light L5 because the foreign substance FS has a property of transmitting the light.

The image processing device 8 of the image acquisition device 1 acquires and stores the image data for the target object S from the imaging device 7 in order to two-dimensionally detect the reflectivity of the reflected light from the target object S by using the above-described properties of the reflected light. By analyzing a brightness distribution of the image data based on the image data stored in the image processing device 8 and searching for a portion in which the intensity of the specularly reflected light is relatively high, it is possible to inspect whether or not the foreign substance FS is present.

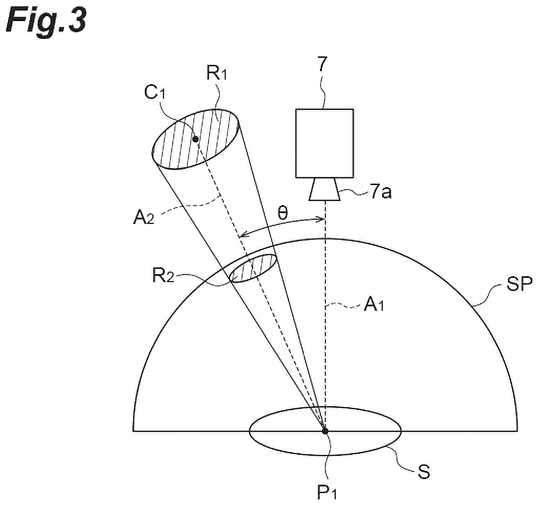

Next, configurations of the illumination device 2 and the imaging device 7 in the image acquisition device 1 will be described in detail with reference to Fig. 3. Fig. 3 is a diagram illustrating disposition of the illumination device 2 with respect to the imaging device 7 and the light emission range of the illumination device 2 in the image acquisition device 1.

The imaging device 7 is disposed at a position and an orientation such that an optical axis $A_1$ of the imaging lens 7a intersects the target object S during the inspection of the target object S. On the other hand, in the illumination device 2, a solid angle $\omega$ viewed from an intersection $P_1$ between the optical axis $A_1$ and the target object S with respect to a light emission range $R_1$ of the light irradiation unit 2a is set to be in a predetermined range, and a straight line $A_2$ connecting a center $C_1$ of the light emission range $R_1$ and the intersection $P_1$ is set to be inclined with respect to the optical axis $A_1$ at an angle $\theta$ in a predetermined angle range. Here, the solid angle $\omega$ means a size of the spread of the light emission range $R_1$ as viewed from the intersection $P_1$, and corresponds to an area of a portion $R_2$ cut out on a spherical surface of radius 1 by a half straight line passing through the light emission range $R_1$ with the intersection $P_1$ as an end point. Specifically, in the image acquisition device 1 according to the present embodiment, the solid angle $\omega$ of the light emission range $R_1$ is set to 0 steradians (sr) or more and 0.15 sr or less, and the angle $\theta$ of the straight line $A_2$ with respect to the optical axis $A_1$ is set to be 2 degrees or more and 120 degrees or less.

Hereinafter, experimental results related to the detection accuracy of the image acquisition device 1 having the above-described configuration will be illustrated. In the experiment, the detection accuracy is evaluated for the target object S in which the foreign substance FS having a known position is disposed, while changing the reflectivity of the foreign substance FS, the solid angle $\omega$, and the parameters of the imaging lens 7a.

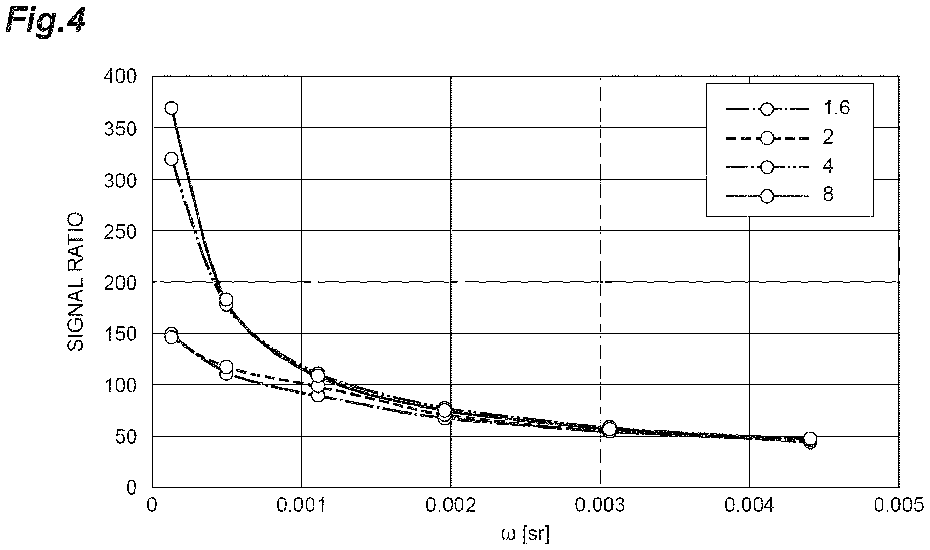

Fig. 4 is a graph illustrating a relationship between the solid angle $\omega$ and a signal ratio indicating the detection accuracy in a case in which the experiment is performed by setting the solid angle $\omega$ to be relatively small and changing an F number of the imaging lens 7a to 1.6, 2, 4, and 8. The signal ratio is a value calculated by dividing the maximum brightness at the position of the foreign substance FS by the average brightness at the position of the target object around the foreign substance FS, and represents a level of the detection accuracy of the foreign substance FS. From these experimental results, in any F number case, the signal ratio is increased as the solid angle $\omega$ is decreased from 0.004 sr to 0 sr. In addition, in a range in which the solid angle $\omega$ is 0.002 sr or less, the signal ratio is higher as the F number is larger, but, from around where the solid angle $\omega$ exceeds 0.002 sr, the change in the signal ratio with respect to the solid angle $\omega$ has almost the same characteristics even in a case in which the F number is changed. This means that the detection accuracy does not depend on observation conditions of the imaging device 7 in a range in which the solid angle $\omega$ exceeds 0.002 sr.

Here, the signal ratio related to the image data acquired by the image acquisition device 1 is estimated by a calculation by a theoretical model. It is assumed that an object-side solid angle of the imaging lens 7a, which is calculated from the F number and the magnification of the imaging lens 7a, is Cam_sr [sr], it is assumed that a solid angle of the illumination device 2 in the light emission range $R_1$ is LS_sr [sr], and it is assumed that the reflectivity of the foreign substance FS is Rs. The intensity of the specularly reflected light from the foreign substance FS in the image data is estimated as a relative value calculated by Rs × Cam_sr. On the other hand, the intensity of the scattered light from the target object S around the foreign substance FS is estimated as a relative value calculated by LS_sr × Cam_sr/2π. Therefore, with this theoretical model, the signal ratio SNr is calculated by the following expression;

$$SNr = 2\pi \times Rs/LS\_sr + 1$$

.

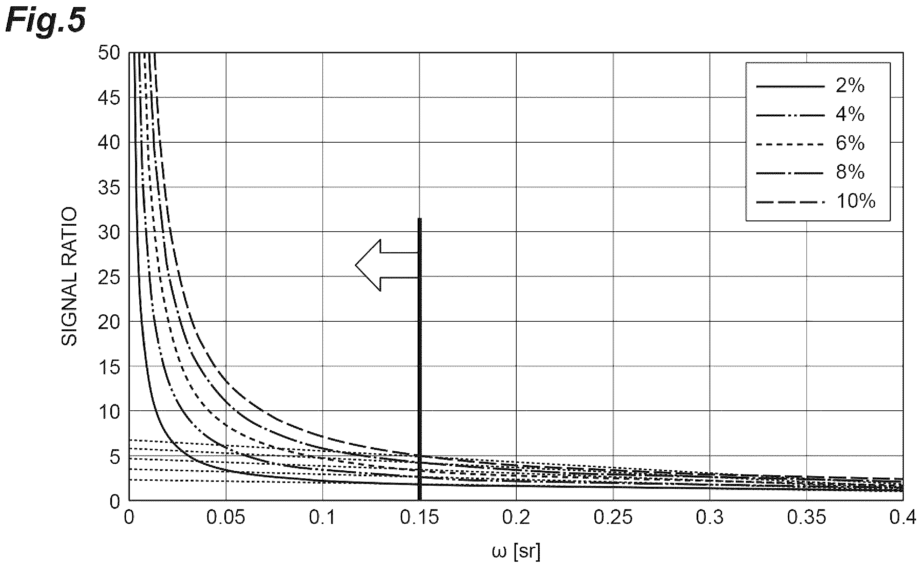

Fig. 5 is a graph illustrating a relationship between the solid angle ω and the signal ratio representing the detection accuracy in a case in which the calculation is performed by the theoretical model by setting the reflectivity of the foreign substance FS in a range of 2% to 10% and changing the solid angle ω in a range of 0 sr to 0.4 sr. From the calculation results, in any reflectivity case, the signal ratio is increased as the solid angle ω is decreased, and, in a case in which the reflectivity is set to be high, the characteristics of the overall signal ratio is also shifted in a direction in which the signal ratio is increased. In particular, in a range in which the solid angle ω exceeds about 0.15 sr, the signal ratio is changed linearly with respect to the solid angle ω, and, in a range in which the solid angle ω is 0 sr or more and about 0.15 sr or less, the signal ratio is changed nonlinearly with respect to the solid angle ω, and a rate of increase in the signal ratio corresponding to the decrease in the solid angle ω rises significantly. That is, the signal ratio SNr is determined only by the reflectivity Rs and the solid angle LS_sr, and this determination is well consistent with the measurement results of the signal ratio characteristics illustrated in Fig. 4. In consideration of such characteristics of the detection accuracy, in the image acquisition device 1 according to the present embodiment, the solid angle ω is set in a range of 0 sr or more and about 0.15 sr or less. With such a setting, the image of the foreign substance FS can be embossed in the image data acquired by the image acquisition device 1.

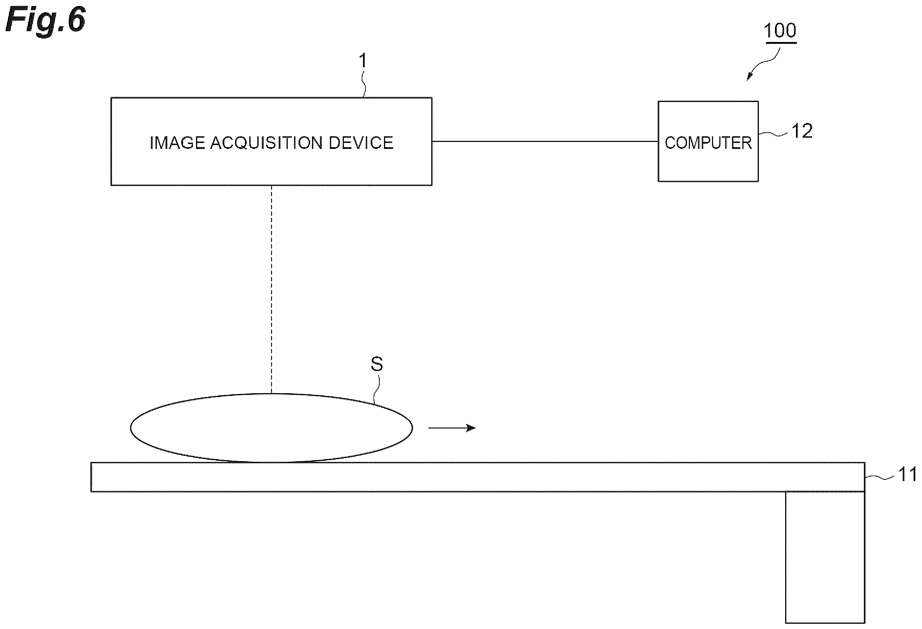

Next, a configuration of an inspection system 100, which is an inspection device according to the embodiment, will be described. Fig. 6 illustrates a schematic configuration of the inspection system 100 according to the embodiment.

The inspection system 100 includes the image acquisition device 1 having the above-described configuration, a transport device 11 such as a belt conveyor that transports the target object S in a predetermined direction, and a computer (inspection processing unit) 12 that performs a computation on the image data output from the image acquisition device 1. The image acquisition device 1 acquires the image data for the target object S transported by the transport device 11 and outputs the acquired image data to the computer 12. In the inspection system 100, the image acquisition device 1 may be controlled to acquire the image data by imaging the target object S in a state in which the target object S is transported by the transport device 11. In the inspection system 100, the image acquisition device 1 may be controlled to acquire the image data by imaging the target object S in a state in which the transport of the target object S is stopped by the transport device 11. In a case in which the imaging is performed in a state in which the target object S is transported, it is preferable to use a line sensor as the imaging device 7, and, in a case in which the imaging is performed in a state in which the transport of the target object S is stopped, it is preferable to use an area sensor as the imaging device 7. In a case in which the imaging is performed in a state in which the target object S is transported, the area sensor may be used as the imaging device 7 to perform the imaging while intermittently lighting the illumination device 2.

The computer 12 has the same hardware configuration as the image processing device 8. That is, the computer 12 is, physically, a computing device that incorporates a CPU or a GPU as a processor, a RAM or a ROM as a recording medium, a communication module, an input/output module, and the like. The computer 12 may acquire the image data from the image processing device 8 via a cable, or may acquire the image data from the image processing device 8 by wireless communication.

Functionally, the computer 12 executes inspection processing for the target object S based on the image data. That is, the computer 12 specifies a brightness difference with reference to the plurality of pieces of image data obtained for the target object S, and determines a range of the foreign substance FS in the target object S based on the specified brightness difference. Then, the computer 12 outputs an inspection result image showing the range of the foreign substance FS determined for one target object S to an output device such as a display. In addition, the computer 12 may output the inspection result image to an external device through a network, a recording medium, and the like.

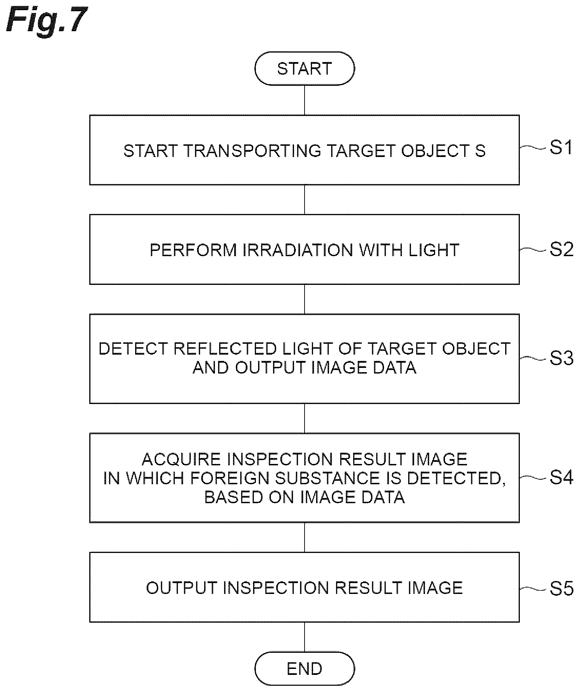

Next, an inspection method for the target object S using the inspection system 100 will be described, and an image acquisition method according to the present embodiment will be described in detail. Fig. 7 is a flowchart illustrating a procedure of the inspection method for the target object S.

First, in a case in which the inspection processing for the target object S is started, the transport of the target object S by the transport device 11 is started (step S1). Then, in a case in which the target object S is transported by the transport device 11 into a range of the irradiation with the light of the illumination device 2, the target object S is irradiated with the light from the illumination device 2 (step S2).

Accordingly, the reflected light generated on the surface of the target object S is incident on the imaging device 7 via the imaging lens 7a, and the two-dimensional image of the reflected light is detected by the imaging device 7, so that the image data is output (S3).

The image data acquired by the imaging device 7 is acquired and stored by the image processing device 8, and then is output to the computer 12 and processed by the computer 12. That is, the computer 12 determines the range of the foreign substance FS on the target object S based on the brightness of each pixel of the image data (step S4).

At last, the computer 12 outputs the inspection result image for the target object S to the output device as the image in which the foreign substance FS present on the target object S is detected (step S5), and terminates the inspection processing of the target object S.

The effects and operations of the present embodiment will be described.

With the image acquisition device 1 according to the present embodiment, in a case in which the foreign substance FS having a property of reflecting the light is present on the target object S, it is possible to cause the specularly reflected light from the foreign substance FS to be efficiently incident on the imaging device 7 via the imaging lens 7a, and it is possible to make the intensity of the specularly reflected light incident from the foreign substance FS sufficiently larger than the intensity of diffused light incident from the target object S. In particular, by setting the solid angle $\omega$ of the light emission range $R_1$ of the light irradiation unit 2a as viewed from the intersection $P_1$ between the optical axis $A_1$ of the imaging lens 7a and the target object S to 0 steradians or more and 0.15 steradians or less, a ratio between the intensity of the specularly reflected light and the intensity of the diffused light can be non-linearly increased. As a result, it is possible to acquire the image data that enables efficient detection of the foreign substance FS present on the target object S and having the property of reflecting the light.

In addition, in the image acquisition device 1, the illumination device 2 has the point-like light emission range of the light irradiation unit 2a. In this case, it is possible to make the intensity of the specularly reflected light incident from the foreign substance FS sufficiently larger than the intensity of the diffused light incident from the target object S, with a simple configuration. As a result, it is possible to acquire the image data that enables the efficient detection of the foreign substance FS present on the target object S and having the property of reflecting the light, with a simple configuration. In addition, in a case in which the illumination device 2 has a configuration in which the plurality of light emitting elements that are the point light sources are incorporated in the light irradiation unit 2a, it is possible to acquire the image data that enables the efficient detection of the foreign substance FS in a wide range of the target object S.

Alternatively, with the inspection system 100 according to the present embodiment, it is possible to efficiently detect the foreign substances FS present on the plurality of target objects S and having the property of reflecting the light while the plurality of target objects S are being transported.

Although various embodiments of the present invention have been described above, the present invention is not limited to the above-described embodiments, and may be modified or applied to other cases without changing the gist described in each claim.

The foreign substance FS, which is the inspection target of the inspection system 100 according to the present embodiment, widely includes a substance having the property of reflecting the light, and includes not only a transparent body such as a plastic film but also a translucent object having color.

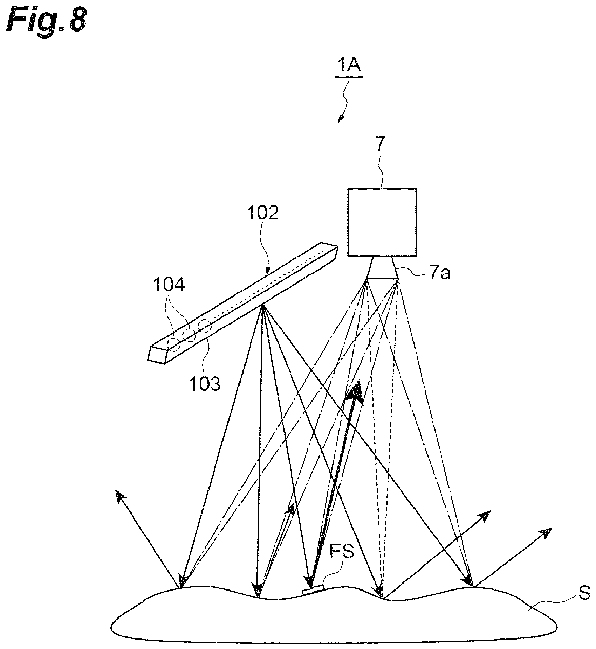

In addition, an illumination device 102 including a light irradiation unit 103 having a linear shape may be used, as in an image acquisition device 1A according to a modification example illustrated in Fig. 8. In the illumination device 102, a plurality of light emitting elements 104, which are point light sources, are disposed along an elongated light emission range of the light irradiation unit 103. With such a configuration, it is possible to acquire the image data that enables the efficient detection of the foreign substance FS having the property of reflecting the light, in a wide range of the target object S. Even in such a configuration, a numerical range of the solid angle $\omega$ of the light emission range $R_1$ and a range (Fig. 3) of the angle $\theta$ of the straight line $A_2$ with respect to the optical axis $A_1$ are set to the same ranges as those in the above-described embodiment. In particular, the image acquisition device 1A is preferably used in combination with the imaging device 7, which is the line sensor, in a case in which the imaging is performed in a state in which the target object S is transported by the transport device 11.

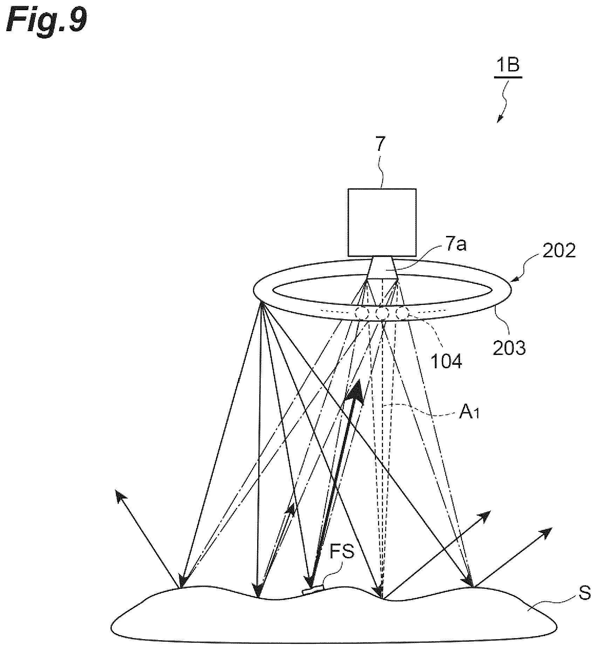

In addition, as in an image acquisition device 1B according to a modification example illustrated in Fig. 9, the illumination device 202 including a light irradiation unit 203 having a ring shape and disposed to surround the optical axis $A_1$ of the imaging lens 7a may be used. In the illumination device 202, a plurality of light emitting elements 104, which are point light sources, are disposed along the ring-shaped light emission range of the light irradiation unit 203. Even in such a configuration, a numerical range of the solid angle $\omega$ of the light emission range $R_1$ and a range (Fig. 3) of the angle $\theta$ of the straight line $A_2$ with respect to the optical axis $A_1$ are set to the same ranges as those in the above-described embodiment. In particular, the image acquisition device 1B is preferably used in combination with the imaging device 7, which is the area sensor, in a case in which the imaging is performed in a state in which the transport of the target object S is stopped.

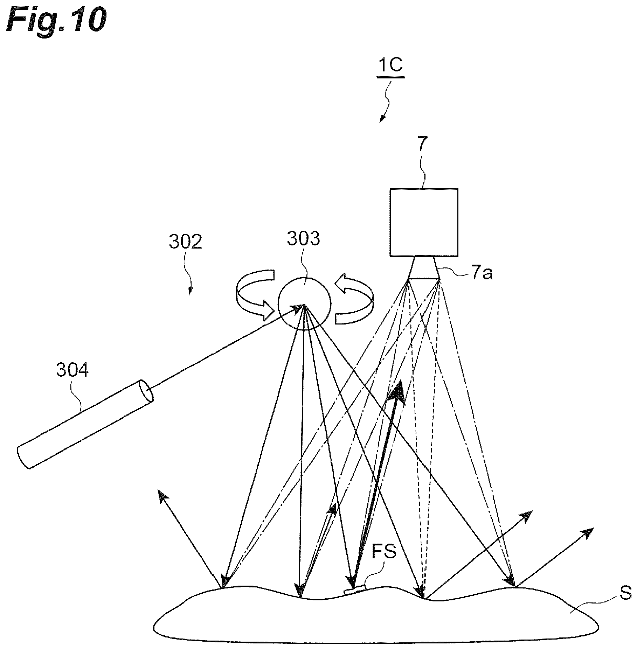

In addition, as in an image acquisition device 1C according to a modification example illustrated in Fig. 10, an illumination device 302 may include a laser light source 304 and an light scanning unit 303 that scans the target object S with the light emitted from the laser light source 304. The light scanning unit 303 forms the light irradiation unit. As the light scanning unit 303, for example, a movable mirror or the like is used. The image acquisition device 1C may have a configuration in which a light irradiation direction of the laser light source 304 itself can be changed, instead of the light scanning unit 303. In this case, a laser scanning optical system that reconstructs an image based on a detection signal for each scanning position of the light may be used as the imaging device 7. Even in such a configuration, a numerical range of the solid angle $\omega$ of the light emission range $R_1$ and a range (Fig. 2) of the angle $\theta$ of the straight line $A_2$ with respect to the optical axis $A_1$ are set to the same ranges as those in the above-described embodiment. With such a configuration, it is possible to acquire the image data that enables the efficient detection of the foreign substance FS having the property of reflecting the light, in a wide range of the target object S.

In addition, the illumination devices 2, 102, 202, and 302 according to the above-described embodiment and respective modification examples may include a combination of various optical members such as a slit as a member that limits a range of the irradiation with the light to the light emission range $R_1$ of the light irradiation unit and limits an angle of the irradiation with the light. In this case, it is possible to set the range of the light irradiation unit with a simple configuration, and it is possible to acquire the image data that enables the efficient detection of the foreign substance FS present on the target object S and having the property of reflecting the light, with a simple configuration.

In addition, in the above-described illumination devices 2, 102, and 202 according to the above-described embodiment and respective modification examples, the light emission range $R_1$ is formed in a point shape, a linear shape, and a ring shape by the plurality of light emitting elements, but the shape thereof may be any curved shape or may be any shape such as a rectangular shape. The plurality of light emitting elements may be continuously disposed or may be discretely disposed. In addition, the illumination devices 2, 102, and 202 may be controlled such that the plurality of light emitting elements are lighted at the same time, may be controlled such that the light emitting elements are partially lighted, or may be controlled such that the light emitting elements are lighted in sequence.

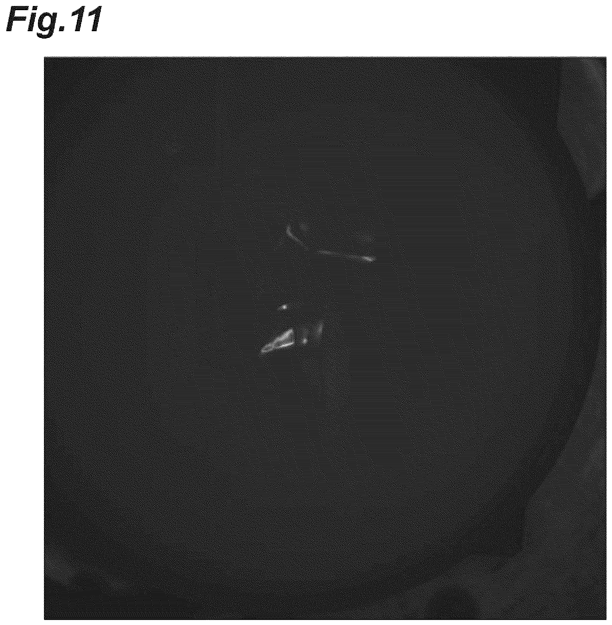

Here, Fig. 11 illustrates an example of the image data acquired by the image acquisition device 1B according to the modification example. This image data is acquired in a case in which a diameter of the light irradiation unit 203 of the illumination device 202 is set to 65 mm, a distance of the light irradiation unit 203 from the target object S is set to 330 mm, the solid angle $\omega$ of the light emission range $R_1$ is set to 0.015 steradians, and the angle $\theta$ of the straight line $A_2$ with respect to the optical axis $A_1$ is set to 5.7 degrees.

In this way, the image acquisition device 1B can acquire the image data in which a portion of the foreign substance FS is more embossed than a portion of the target object S.

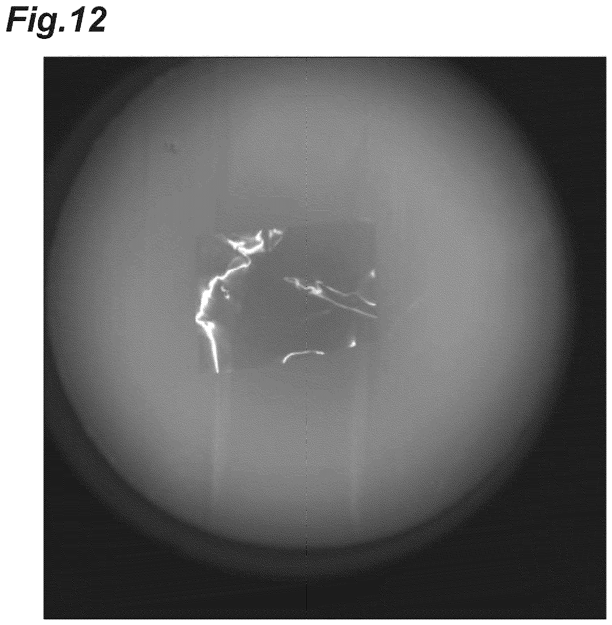

In addition, Fig. 12 illustrates an example of image data acquired by an image acquisition device according to a comparative example. In this comparative example, the image data is acquired in a case in which the diameter of the light irradiation unit 203 of the illumination device 202 is set to 65 mm, the distance of the light irradiation unit 203 from the target object S is set to 70 mm, the solid angle $\omega$ of the light emission range $R_1$ is set to 0.29 steradians, and the angle $\theta$ of the straight line $A_2$ with respect to the optical axis $A_1$ is set to 25 degrees. In the image data acquired in such a modification example, a difference between the brightness of the portion of the foreign substance FS and the brightness of the portion of the target object S is eliminated. As a result, it is difficult to detect the presence of the foreign substance FS from the image data.

In addition, in the image acquisition devices 1, 1A, 1B, and 1C described above, the angle $\theta$ of the straight line $A_2$ with respect to the optical axis $A_1$ may be set to be 2 degrees or more and 90 degrees or less. By the setting as described above, in a case in which the target object S having a planar shape is set as the inspection target, it is possible to cause the specularly reflected light from the foreign substance FS on the target object S, which is generated based on the light from the light irradiation unit of the illumination devices 2, 102, 202, and 302, to be efficiently incident by the imaging device 7, and it is possible to further increase a ratio of the intensity of the specularly reflected light incident from the foreign substance FS to the intensity of the diffused light incident from the target object S. As a result, it is possible to acquire the image data that enables the efficient detection of the foreign substance FS on the target object S having a planar shape.

In the above-described embodiment, it is preferable that the straight line connecting the center of the light irradiation unit and the intersection is set to be inclined by 2 degrees or more and 90 degrees or less with respect to the optical axis of the imaging lens. By the setting as described above, it is possible to cause the specularly reflected light from the object on the target object having a planar shape, which is generated based on the light from the light irradiation unit, to be more efficiently incident on the imaging device, and it is possible to further increase a ratio of the intensity of the specularly reflected light incident from the object to the intensity of the diffused light incident from the target object. As a result, it is possible to acquire the image data that enables efficient detection of the object present on the target object of a planar shape and having the property of reflecting the light.

In addition, in the above-described embodiment, it is also preferable that the light irradiation device includes a point light source. In this case, it is possible to make the intensity of the specularly reflected light incident from the object sufficiently larger than the intensity of the diffused light incident from the target object, with a simple configuration. As a result, it is possible to acquire the image data that enables the efficient detection of the object present on the target object and having the property of reflecting the light, with a simple configuration.

Further, in the above-described embodiment, it is also preferable that the light irradiation device includes the light irradiation unit in which a plurality of the point light sources are disposed. In this manner, it is possible to acquire the image data that enables the efficient detection of the object having the property of reflecting the light, in a wide range of the target object.

Furthermore, in the above-described embodiment, it is also preferable that the light irradiation device includes a light scanning unit that scans the target object with the light. In this manner, it is possible to acquire the image data that enables the efficient detection of the object having the property of reflecting the light, in a wide range of the target object.

In addition, in the above-described embodiment, it is preferable that the light irradiation device includes a member that limits a range of the irradiation with the light to the range of the light irradiation unit. In this case, it is possible to set the range of the light irradiation unit with a simple configuration, and it is possible to acquire the image data that enables the efficient detection of the object present on the target object and having the property of reflecting the light, with a simple configuration.

The image acquisition device according to the embodiment is [1] "An image acquisition device including: a light irradiation device that irradiates a target object with light from a range of a light irradiation unit; and an imaging device that detects light that is specularly reflected by the target object in the light, via an imaging lens, in which a straight line connecting a center of the light irradiation unit and an intersection between an optical axis of the imaging lens and the target object is set to be inclined by 2 degrees or more and 120 degrees or less with respect to the optical axis, and a solid angle of the light irradiation unit as viewed from the intersection is set to 0 steradians or more and 0.15 steradians or less".

The image acquisition device according to the embodiment may be [2] "The image acquisition device according to [1], in which the straight line connecting the center of the light irradiation unit and the intersection is set to be inclined by 2 degrees or more and 90 degrees or less with respect to the optical axis of the imaging lens".

The image acquisition device according to the embodiment may be [3] "The image acquisition device according to [1] or [2], in which the light irradiation device includes a point light source".

The image acquisition device according to the embodiment may be [4] "The image acquisition device according to [3], in which the light irradiation device includes the light irradiation unit in which a plurality of the point light sources are disposed".

The image acquisition device according to the embodiment may be [5] "The image acquisition device according to any one of [1] to [4], in which the light irradiation device includes a light scanning unit that scans the target object with the light".

The image acquisition device according to the embodiment may be [6] "The image acquisition device according to any one of [1] to [5], in which the light irradiation device includes a member that limits a range of the irradiation with the light to the range of the light irradiation unit".

The inspection device according to the embodiment is [7] "An inspection device including: the image acquisition device according to any one of [1] to [6]; a transport device that transports the target object in a predetermined direction; and an inspection processing unit that inspects the target object based on data output from the image acquisition device".

The image acquisition method according to the embodiment is [8] "An image acquisition method including: a light irradiation step of using a light irradiation device to irradiate a target object with light from a range of a light irradiation unit; and an imaging step of using an imaging device to detect light that is specularly reflected by the target object in the light, via an imaging lens, in which a straight line connecting a center of the light irradiation unit and an intersection between an optical axis of the imaging lens and the target object is set to be inclined by 2 degrees or more and 120 degrees or less with respect to the optical axis, and a solid angle of the light irradiation unit as viewed from the intersection is set to 0 steradians or more and 0.15 steradians or less".

**Reference Signs List**

1, 1A, 1B, 1C: image acquisition device
2, 102, 202, 302: illumination device (light irradiation device)
2a, 103, 203: light irradiation unit
303: light scanning unit (light irradiation unit)
104: light emitting element (point light source)
7: imaging device
7a: imaging lens
11: transport device
12: computer (inspection processing unit)
100: inspection system (inspection device)
$A_1$: optical axis
$A_2$: straight line
$C_1$: center
$P_1$: intersection
$\theta$: angle
S: target object
FS: foreign substance

**Claims**

1. An image acquisition device comprising:

   a light irradiation device that irradiates a target object with light from a range of a light irradiation unit; and
   an imaging device that detects light that is specularly reflected by the target object in the light, via an imaging lens,

wherein a straight line connecting a center of the light irradiation unit and an intersection between an optical axis of the imaging lens and the target object is set to be inclined by 2 degrees or more and 120 degrees or less with respect to the optical axis, and

a solid angle of the light irradiation unit as viewed from the intersection is set to 0 steradians or more and 0.15 steradians or less.

2. The image acquisition device according to Claim 1, wherein the straight line connecting the center of the light irradiation unit and the intersection is set to be inclined by 2 degrees or more and 90 degrees or less with respect to the optical axis of the imaging lens.

3. The image acquisition device according to Claim 1 or 2,
wherein the light irradiation device includes a point light source.

4. The image acquisition device according to Claim 3, wherein the light irradiation device includes the light irradiation unit in which a plurality of the point light sources are disposed.

5. The image acquisition device according to any one of Claims 1 to 4,
wherein the light irradiation device includes a light scanning unit that scans the target object with the light.

6. The image acquisition device according to any one of Claims 1 to 5,
wherein the light irradiation device includes a member that limits a range of the irradiation with the light to the range of the light irradiation unit.

7. An inspection device comprising:

the image acquisition device according to any one of Claims 1 to 6;
a transport device that transports the target object in a predetermined direction; and
an inspection processing unit that inspects the target object based on data output from the image acquisition device.

8. An image acquisition method comprising:

a light irradiation step of using a light irradiation device to irradiate a target object with light from a range of a light irradiation unit; and
an imaging step of using an imaging device to detect light that is specularly reflected by the target object in the light, via an imaging lens,

wherein a straight line connecting a center of the light irradiation unit and an intersection between an optical axis of the imaging lens and the target object is set to be inclined by 2 degrees or more and 120 degrees or less with respect to the optical axis, and

a solid angle of the light irradiation unit as viewed from the intersection is set to 0 steradians or more and 0.15 steradians or less.

9. The image acquisition method according to Claim 8, wherein the straight line connecting the center of the light irradiation unit and the intersection is set to be inclined by 2 degrees or more and 90 degrees or less with respect to the optical axis of the imaging lens.

10. The image acquisition method according to Claim 8 or 9, wherein the light irradiation device includes a point light source.

11. The image acquisition method according to Claim 10,
wherein the light irradiation device includes the light irradiation unit in which a plurality of the point light sources are disposed.

12. The image acquisition method according to any one of Claims 8 to 11,
wherein the light irradiation device includes a light scanning unit that scans the target object with the light.

13. The image acquisition method according to any one of Claims 8 to 12,
wherein the light irradiation device includes a member that limits a range of the irradiation with the light to the range of the light irradiation unit.

*Fig.1*

# Fig.2

**Fig.3**

# Fig.4

# Fig.5

**Fig.6**

EP 4 597 082 A1

# Fig.7

```
              ┌─────────────┐
              │    START    │
              └──────┬──────┘
                     │
    ┌────────────────┴────────────────┐
    │  START TRANSPORTING TARGET OBJECT S  │─── S1
    └────────────────┬────────────────┘
                     │
    ┌────────────────┴────────────────┐
    │   PERFORM IRRADIATION WITH LIGHT  │─── S2
    └────────────────┬────────────────┘
                     │
    ┌────────────────┴────────────────┐
    │  DETECT REFLECTED LIGHT OF TARGET OBJECT │─── S3
    │        AND OUTPUT IMAGE DATA      │
    └────────────────┬────────────────┘
                     │
    ┌────────────────┴────────────────┐
    │   ACQUIRE INSPECTION RESULT IMAGE │
    │ IN WHICH FOREIGN SUBSTANCE IS DETECTED, │─── S4
    │         BASED ON IMAGE DATA       │
    └────────────────┬────────────────┘
                     │
    ┌────────────────┴────────────────┐
    │   OUTPUT INSPECTION RESULT IMAGE  │─── S5
    └────────────────┬────────────────┘
                     │
              ┌──────┴──────┐
              │     END     │
              └─────────────┘
```

**Fig.8**

*Fig.9*

## Fig.10

Fig.11

Fig.12

## INTERNATIONAL SEARCH REPORT

| International application No. |
| --- |
| **PCT/JP2023/028774** |

| A. | CLASSIFICATION OF SUBJECT MATTER |
| --- | --- |

*G01N 21/84*(2006.01)i; *G01N 21/89*(2006.01)i
FI:    G01N21/84 E; G01N21/89 Z

According to International Patent Classification (IPC) or to both national classification and IPC

| B. | FIELDS SEARCHED |
| --- | --- |

Minimum documentation searched (classification system followed by classification symbols)

    G01N21/00-21/958

Documentation searched other than minimum documentation to the extent that such documents are included in the fields searched

    Published examined utility model applications of Japan 1922-1996
    Published unexamined utility model applications of Japan 1971-2023
    Registered utility model specifications of Japan 1996-2023
    Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2014/0160472 A1 (LEVESQUE, M.) 12 June 2014 (2014-06-12) <br>     paragraphs [0001], [0055]-[0120], fig. 2-9 | 1-13 |
| X | JP 2000-28536 A (NIDEK CO., LTD.) 28 January 2000 (2000-01-28) <br>     paragraphs [0035]-[0048], fig. 1-3 | 1-6, 8-13 |
| Y |     paragraphs [0035]-[0048], fig. 1-3 | 7 |
| Y | JP 2020-148661 A (OMRON CORP.) 17 September 2020 (2020-09-17) <br>     paragraph [0018], fig. 1 | 7 |
| A | JP 2021-179331 A (NIPPON STEEL CORP.) 18 November 2021 (2021-11-18) | 1-13 |
| A | JP 2021-196256 A (MACHINE VISION LIGHTING INC.) 27 December 2021 (2021-12-27) | 1-13 |
| A | WO 2020/174596 A1 (NEC CORP.) 03 September 2020 (2020-09-03) | 1-13 |

| ☐ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 September 2023** | **10 October 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT
### Information on patent family members

International application No.

**PCT/JP2023/028774**

| Patent document cited in search report | | | Publication date (day/month/year) | Patent family member(s) | | | Publication date (day/month/year) |
|---|---|---|---|---|---|---|---|
| US | 2014/0160472 | A1 | 12 June 2014 | WO | 2013/027083 | A1 | |
| | | | | CA | 2842544 | A1 | |
| JP | 2000-28536 | A | 28 January 2000 | US | 6222624 | B1 | |
| | | | | column 12, line 20 to column 15, line 2, fig. 6-8 | | | |
| | | | | EP | 930498 | A2 | |
| JP | 2020-148661 | A | 17 September 2020 | WO | 2020/184530 | A1 | |
| JP | 2021-179331 | A | 18 November 2021 | (Family: none) | | | |
| JP | 2021-196256 | A | 27 December 2021 | US | 2022/0170863 | A1 | |
| | | | | WO | 2021/255793 | A1 | |
| | | | | EP | 4166933 | A1 | |
| | | | | KR | 10-2021-0157400 | A | |
| | | | | CN | 114144661 | A | |
| | | | | JP | 6799272 | B1 | |
| WO | 2020/174596 | A1 | 03 September 2020 | US | 2022/0141369 | A1 | |
| | | | | EP | 3933386 | A1 | |

Form PCT/ISA/210 (patent family annex) (January 2015)

23

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021189071 A